# EUROPÄISCHE PATENTSCHRIFT

(11) **EP 2 852 340 B1**
(45) Veröffentlichungstag und Bekanntmachung des Hinweises auf die Patenterteilung: **07.12.2016**
(21) Anmeldenummer: 13725107.0
(22) Anmeldetag: 14.05.2013
(51) Int. Cl.: A61F 7/12, A61B 17/221, A61M 27/00, A61B 17/3207, A61F 7/00, A61B 17/32, A61B 17/22

(54) **MEDIZINISCHES SYSTEM ZUM ENDOVASKULÄREN TEMPERIEREN VON BLUT**
MEDICAL SYSTEM FOR ENDOVASCULAR TEMPERATURE CONTROL OF BLOOD
SYSTÈME MÉDICAL PERMETTANT LA RÉGULATION ENDOVASCULAIRE DE LA TEMPÉRATURE DU SANG

(30) Priorität: 22.05.2012 DE 102012104381
(43) Veröffentlichungstag der Anmeldung: 01.04.2015
(73) Patentinhaber: Acandis GmbH & Co. KG, 76327 Pfinztal (DE)
(72) Erfinder: CATTANEO, Giorgio, 76199 Karlsruhe (DE)
(74) Vertreter: Kilchert, Jochen
(86) Internationale Anmeldenummer: PCT/EP2013/059925
(87) Internationale Veröffentlichungsnummer: WO 2013/174676

(56) Entgegenhaltungen:
- US-A1- 2002 103 472
- US-A1- 2005 085 849
- US-A1- 2006 009 785
- US-A1- 2007 250 050
- US-A1- 2011 319 754
- US-B1- 6 537 271
- US-B1- 6 702 783
- US-B1- 8 052 701

## Beschreibung

Die Erfindung betrifft ein medizinisches System zum endovaskulären Temperieren von Blut und zur Behandlung, insbesondere zur Rekanalisation eines Blutgefäßes sowie einen medizinischen Katheter.

Rekanalisationsvorrichtungen ohne die Möglichkeit der Temperierung werden im medizinischen Bereich zur Wiederherstellung des Blutflusses in Blutgefäßen eingesetzt. Der Blutfluss kann beispielsweise durch Blutgerinnsel, insbesondere Thromben, unterbrochen sein, wodurch sich eine Unterversorgung nachgeordneter Gewebeareale mit Nährstoffen und Sauerstoff ergibt. Um die Versorgung wiederherzustellen, werden die Blutgerinnsel mechanisch und/oder medikamentös entfernt.

US 2005/0085849 A1 beschreibt eine derartige Rekanalisationsvorrichtung, die eine mechanische Entfernung von Thromben ermöglicht. Dazu weist die Vorrichtung einen Führungsdraht mit einem distalen Abschnitt auf, der sich beim Freisetzen in einem Blutgefäß spiralförmig bzw. schraubenförmig radial aufweitet. Mit der sich ergebenden korkenzieherartigen Struktur, die mittels eines Katheters distal vom Blutgerinnsel positioniert wird, erfolgt eine Entfernung des Blutgerinnsels durch Zurückziehen des Führungsdrahtes.

US 2007/0250050 A1 beschreibt ein Gerät zur Kryobehandlung. Hierzu weist ein Kühlungskatheter einen Ballon mit einer Ballonumhüllung auf, wobei die Ballonumhüllung mit einem Kühlmittelrohr und einem Führungsdrahtrohr verbunden sein kann.

Eine Komplikation bei der Rekanalisation eines Blutgefäßes, beispielsweise nach einem Schlaganfall, ist die Blutung. Dies hängt damit zusammen, dass die nekrotischen Bereiche distal vom Gefäßverschluss Gefäße mit degenerierter Gefäßwand aufweisen. Wenn es bei der Rekanalisation zu einem plötzlichen Blutdurchfluss kommt, der mit der Wiederherstellung des arteriellen Blutdruckes einhergeht, können distale Gefäße beschädigt werden, was zu Blutungen führen kann. Dieses Problem tritt gerade bei mechanischen Rekanalisationssystemen auf, die zu einer schnellen Rekanalisation des betroffenen Gefäßes führen.

Der Erfindung liegt die Aufgabe zu Grunde, ein medizinisches System anzugeben, das die Nebenwirkungen bei der mechanischen Rekanalisation von Gefäßverschlüssen verringert und das darüber hinaus zur wirksamen Behandlung von Schlaganfällen geeignet ist.

Erfindungsgemäß wird diese Aufgabe durch ein medizinisches System mit den Merkmalen des Anspruchs 1 alternativ durch ein System mit den Merkmalen des Anspruchs 14 gelöst.

Die Erfindung beruht auf dem Gedanken, ein medizinisches System zum endovaskulären Temperieren von Blut und zur Behandlung, insbesondere zur Rekanalisation eines Blutgefäßes anzugeben.

Das System weist eine Zuführung und eine radial komprimierbare Behandlungseinrichtung, insbesondere Rekanalisationseinrichtung auf, die im komprimierten Zustand in der Zuführung längsbeweglich angeordnet und durch Entlassen aus der Zuführung zur Behandlung, insbesondere zur Rekanalisation des Blutgefäßes radial expandierbar ist. Das System weist ferner ein Temperierelement zum Temperieren von Blut auf. Die Behandlungseinrichtung, insbesondere die Rekanalisationseinrichtung, ist distal zum Temperierelement positionierbar derart, dass im Gebrauch durch das Temperierelement temperiertes Blut zur Behandlungsstelle, insbesondere zu Rekanalisationsstelle im Blutgefäß strömt.

Die Erfindung kombiniert die Funktionen mechanischer Behandlungssysteme, insbesondere mechanischer Rekanalisationssysteme mit den Vorteilen der endovaskulären Hypothermie in einem einzigen System bzw. Gerät. Damit ist zum Kühlen, bzw. allgemein zum Temperieren, und zum Behandeln, insbesondere zum Rekanalisieren kein Gerätewechsel nötig. Beide Vorgänge werden erfindungsgemäß mit demselben System bzw. Gerät durchgeführt, wodurch die Wirksamkeit der Behandlung verbessert wird. Das Temperieren und die Behandlung, insbesondere das Rekanalisieren können dabei zeitgleich oder zeitlich versetzt erfolgen.

Die Erfindung ist nicht nur zum Kühlen, sondern auch zum Erwärmen von Blut (Hyperthermie) geeignet, also allgemeinen zum Temperieren von Blut. Ohne die Erfindung darauf einzuschränken, wird das System nachfolgend im Zusammenhang mit der bevorzugten Blutkühlung beschrieben.

Mit dem kombinierten Hypothermie-/Rekanalisationssystem wird die Bildung von Hämatomen reduziert, die auftreten können, wenn es bei der mechanischen Rekanalisation zu Gefäßblutungen kommt. Dabei erfolgt erfindungsgemäß die Kühlung des Blutes und die mechanische Aufweitung des Gefäßes durch ein und dasselbe System, ohne dass dafür während der Behandlung ein Gerätewechsel erforderlich ist.

Darüber hinaus nutzt das erfindungsgemäße System weitere positive Effekte der Hypothermie, die gerade im Zusammenhang mit der integrierten Rekanalisationsfunktion besonders zum Tragen kommen. So ist die endovaskuläre Hypothermie besonders zur Behandlung von Schlaganfällen geeignet, da diese zur Verlängerung des Zeitfensters bei der Behandlung beiträgt. Im Gegensatz zur Lyse ist die Hypothermie auch zur Behandlung von hämorrhagischen Schlaganfällen geeignet. Die Hypothermie kann deshalb als eine der ersten Behandlungsmaßnahmen unabhängig von der Art des Schlaganfalls (ischämisch oder hämorrhagisch) eingesetzt werden. Konkret hat die endovaskuläre Hypothermie den Vorteil, dass der Wärmeübergang von Blut auf das Temperiermedium direkt im Gefäß erfolgt. Die endovaskuläre Hypothermie bewirkt damit einen besonders wirkungsvollen Wärmeübergang. Außerdem kann eine sehr lokale Kühlwirkung erzeugt werden, die wiederum im Zusammenhang mit der integrierten Rekanalisationsfunktion die gezielte Behandlung spezieller lokal begrenzter Körperbereiche ermöglicht.

Die Erfindung ist nicht auf Rekanalisationssysteme wie Stents beschränkt, sondern ermöglicht die Kombination der Temperierfunktion allgemein mit mechanischen Behandlungssytemen, die endovaskulär eingesetzt werden. Konkret umfasst im Rahmen der Erfindung eine radial komprimierbare und expandierbare Behandlungseinrichtung wie Flow Diverter, bspw. Aneurysmastents oder Stentgrafts, Thrombektomieeinrichtungen oder Okklusionseinrichtungen. Als konkretes Beispiele eines Aneurysmastents wird auf den von der Anmelderin hergestellten und vertriebenen Stent Neuro Closed und als konkretes Beispiel einer Thrombektomieeinrichtung auf das ebenfalls von der Anmelderin hergestellte und vertriebene Device Aperio verwiesen. Eine im Rahmen der Erfindung verwendbare Okklusionseinrichtung sind Aneurysmacoils, die nach dem Freisetzen aus der gestreckten Form in eine gewundene Form übergehen. Die gestreckte Form entspricht dem radial komprimierten Zustand und die gewundene Form dem radial expandierten Zustand.

Besonders bevorzugte Behandlungssysteme sind Rekanalisationssysteme wie bspw. Stents.

Zwar sind bereits medizinische Geräte für die endovaskuläre Hypothermie, beispielsweise Ballonkatheter bekannt. Ein derartiger Ballonkatheter ist in US 6,702,783 beschrieben. Die bekannten Geräte dienen aber ausschließlich der Blutkühlung und nicht der Rekanalisation von Blutgefäßen.

Außerdem sind die bekannten Hypothermiegeräte so groß dimensioniert, dass diese nicht zur Behandlung distal gelegener Gefäße, beispielsweise im zerebralen Bereich einsetzbar sind.

Das erfindungsgemäße System zum endovaskulären Temperieren von Blut und zur Behandlung, insbesondere zur Rekanalisation eines Blutgefäßes ist demgegenüber flexibel einsetzbar und ermöglicht auch die Behandlung kleinlumiger Gefäße. Dazu weist das erfindungsgemäße System eine Behandlungseinrichtung, insbesondere Rekanalisationseinrichtung und ein gesondertes Temperierelement auf, wobei die Behandlungseinrichtung, insbesondere Rekanalisationseinrichtung im Wesentlichen unabhängig vom Temperierelement im Blutgefäß positionierbar ist. Die sich daraus ergebende Funktionstrennung innerhalb ein- und desselben medizinischen Systems ermöglicht die Optimierung der Funktion der jeweiligen Komponente unabhängig von der Funktion der jeweils anderen Komponente. So ist es beispielsweise möglich, die Behandlungseinrichtung, insbesondere die Rekanalisationseinrichtung so zu dimensionieren, dass diese in Gefäßen mit kleinen Durchmessern expandiert werden kann.

Das Temperierelement ist im Gebrauch, d.h. bei freigesetzter, expandierter Behandlungseinrichtung, insbesondere Rekanalisationseinrichtung proximal von dieser angeordnet. Da sich in aller Regel der Gefäßdurchmesser von distal nach proximal vergrößert, kann das Temperierelement im Gebrauch einen größeren Durchmesser als die Behandlungseinrichtung, insbesondere die Rekanalisationseinrichtung aufweisen. Damit kann die Oberfläche, die den Wirkungsgrad des Temperierelements mitbestimmt, für den Wärmeübergang optimiert werden, ohne dass es dabei zu Einschränkungen bei der Dimensionierung der Behandlungseinrichtung, insbesondere Rekanalisationseinrichtung kommt, da diese unabhängig vom Temperierelement ist.

Bevorzugte Ausführungen der Erfindung sind in den Unteransprüchen angegeben.

Die nachfolgend im Zusammenhang mit der Rekanalisationseinrichtung beschriebenen Merkmale und Vorteile der erfindungsgemäßen Ausführungen werden auch allgemein im Zusammenhang mit der Behandlungseinrichtung offenbart und beansprucht. Dies gilt für die gesamte Anmeldung.

So kann die Rekanalisationseinrichtung eine radial expandierbare und radial komprimierbare Gitterstruktur aufweisen, die im expandierten Zustand von Blut durchströmbar ist. Derartige Rekanalisationseinrichtungen sind an sich bekannt und haben im Zusammenhang mit dem kombinierten Temperier-/Rekanalisationssystem den Vorteil, dass die Bluttemperierung, insbesondere die Blutkühlung und die Rekanalisation des Blutgefäßes gleichzeitig erfolgen kann.

Generell kann mit dem System das Blut vor und nach der Rekanalisation temperiert werden. Die Temperierung während der Rekanalisation erfordert eine im expandierten Zustand von Blut durchströmbare Rekanalisationseinrichtung, wie beispielsweise die vorstehend erwähnte Gitterstruktur.

Zur gemeinsamen Handhabung und Betätigung der Rekanalisationseinrichtung und des Temperierelements kann die Zuführung einen Kanal für die Rekanalisationseinrichtung aufweisen, der sich entlang wenigstens einer Temperierleitung erstreckt, die von einem Temperiermedium durchströmbar und mit dem Temperierelement verbunden ist.

Bei einer bevorzugten Ausführung ist eine Zuführleitung im Kanal längsbeweglich angeordnet, wobei die Rekanalisationseinrichtung in der Zuführleitung längsbeweglich angeordnet ist. Durch die Zuführleitung wird eine Teleskopierfunktion erreicht, mit der sich der Abstand zwischen dem Temperierelement und der Rekanalisationseinrichtung einfach einstellen lässt.

Dadurch können patientenspezifischen Gegebenheiten wie Gefäßdurchmesser, Gefäßkrümmung usw. berücksichtigt und entfernte, kleinlumige Gefäßbereiche durch die Rekanalisationseinrichtung erreicht werden.

Dazu wird das Temperierelement, das einen größeren Durchmesser als die Rekanalisationseinrichtung aufweist, an einer Stelle platziert, an der das Blutgefäß einen ausreichend großen Durchmesser aufweist. Für andere Anwendungen kann der Durchmesser des Temperierelements dem Durchmesser der Rekanalisationseinrichtung entsprechen oder kleiner sein. Die Zuführleitung wird mit oder ohne Führungsdraht aus dem Kanal in distaler Richtung in die engen Gefäßbereiche bis zur Rekanalisierungsstelle vorgeschoben, so dass die Rekanalisationseinrichtung positioniert werden kann. Dazu wird die Rekanalisationseinrichtung in der Zuführleitung bis zur Rekanalisierungsstelle vorgeschoben, die nach korrekter Positionierung der Rekanalisationseinrichtung zurückgezogen wird. Dadurch wird die Rekanalisationseinrichtung entlassen und expandiert in radialer Richtung, wobei der freie Strömungsquerschnitt des Gefäßes wiederhergestellt bzw. vergrößert wird.

Alternativ kann die Rekanalisationseinrichtung unmittelbar im Kanal längsbeweglich angeordnet sein. Diese Ausführung ist einfach aufgebaut und lässt sich einfach herstellen. Außerdem kann ein kleiner Kanaldurchmesser verwendet werden.

Vorzugsweise sind wenigstens zwei Temperierleitungen mit dem Temperierelement verbunden, wodurch ein kontinuierlicher Vor- und Rücklauf des Temperiermediums, d.h. eine kontinuierliche Temperierung ermöglicht wird. Alternativ kann eine einzige Temperierleitung für einen pulsatilen Vor- und Rücklauf des Temperiermediums mit dem Temperierelement verbunden sein. Damit wird der Aufbau vereinfacht.

Wenn die Temperierleitung für den Vorlauf innerhalb der Temperierleitung für den Rücklauf angeordnet ist, wird das zum Temperierelement geführte Temperiermedium gegen das umgebende Blut thermisch isoliert.

Die Versorgung des Temperierelements kann dadurch erreicht werden, dass die Temperierleitung für den Vorlauf in distaler Richtung über die Temperierleitung für den Rücklauf vorsteht, wobei ein proximales Ende des Temperierelements mit der Temperierleitung für den Rücklauf und ein distales Ende des Temperierelements mit der Temperierleitung für den Vorlauf verbunden, insbesondere jeweils fluiddicht verbunden ist. Damit entsteht ein an den axialen Enden fluiddicht abgeschlossener Aufnahmeraum zwischen den distalen Enden der Temperierleitung für den Vorlauf und der Temperierleitung für den Rücklauf, der mit dem Temperiermedium gefüllt werden kann.

Wenn wenigstens eine Austrittsöffnung in der Temperierleitung für den Vorlauf am distalen Ende des Temperierelements angeordnet ist, strömt das Temperiermedium am distalen Ende des Temperierelements aus und wird zum proximalen Ende des Temperierelements und von dort in die Temperierleitung für den Rücklauf zurückgeführt. Damit wird eine möglichst lange Temperier- bzw. Wärmetauscherstrecke erreicht.

Alternativ kann die einzige Temperierleitung für den Vor- und Rücklauf in distaler Richtung über den Kanal vorstehen, wobei ein proximales Ende des Temperierelements mit der einzigen Temperierleitung und ein distales Ende des Temperierelements mit dem Kanal verbunden, insbesondere fluiddicht verbunden ist. Die Vorteile dieser Ausführung sind im Zusammenhang mit der mehrlumigen Zuführung des Temperiermediums beschrieben.

Vorzugsweise bildet das Temperierelement einen Temperierballon, insbesondere einen profilierten Temperierballon, der den Vorteil hat, dass die Expansion durch den Druck des Temperiermediums erreicht wird.

Ein einfacher Aufbau des Systems wird erreicht, wenn die wenigstens eine Temperierleitung, insbesondere beide Temperierleitungen, und der Kanal für die Rekanalisationseinrichtung koaxial angeordnet sind.

Es hat sich gezeigt, dass eine gute Positionierbarkeit des Temperierelements und der Behandlungseinrichtung, insbesondere Rekanalisationseinrichtung erreicht wird, wenn der maximal einstellbare Abstand zwischen dem distalen Ende der Zuführleitung und dem distalen Ende der Zuführung, insbesondere des Kanals von 10 cm bis 30 cm, insbesondere von 15 cm bis 25 cm beträgt.

Die Offenbarung betrifft ferner einen medizinischen Katheter aufweisend eine Hauptleitung mit wenigstens drei Arbeitslumen, wobei ein erstes Arbeitslumen exzentrisch bezogen auf die Hauptleitung und ein zweites und drittes Arbeitslumen konzentrisch zueinander angeordnet sind. Ein solcher Katheter ist zur Verwendung in einem System nach einem der Ansprüche 1-12 oder 14 geeignet und wird im Zusammenhang mit diesem System, wie es in den Ansprüchen und der Beschreibung erläutert ist, offenbart. Der Katheter wird unabhängig von dem System beschrieben und ist auch für andere Einsatzzwecke geeignet.

Der Katheter hat den Vorteil, dass durch die exzentrische Anordnung des ersten Arbeitslumens das Volumen eines der beiden anderen Arbeitslumen, insbesondere des zweiten Arbeitslumens vergrößert wird. Dieser Vorteil kommt besonders bei dem erfindungsgemäßen System zum Tragen, wenn das erste Arbeitslumen die Zuführung für die Rekanalisationseinrichtung und das zweite Arbeitslumen eine der Temperierleitungen, insbesondere die Temperierleitung für den Vorlauf, bildet. Dann wird der Strömungsquerschnitt für das Temperiermedium vergrößert.

Wenn die Temperierleitung für den Vorlauf radial innen angeordnet ist, hat dies den Vorteil, dass eine optimale Isolierung des Temperiermediums in der Temperierleitung für den Vorlauf erreicht wird. Dabei umgibt die Temperierleitung für den Rücklauf radial außen einen Umfangsabschnitt der Temperierleitung für den Vorlauf. Ein weiterer bzw. der verbleibende Umfangsabschnitt der Temperierleitung für den Vorlauf ist durch das exzentrisch angeordnete erste Arbeitslumen, insbesondere den Kanal, isoliert.

Gegenüber Systemen, bei denen alle Arbeitslumen konzentrisch angeordnet sind, hat der erfindungsgemäße Katheter den Vorteil, dass die einzelnen Arbeitslumen entlang der Katheterlänge positionsfest sind. Dies kann bspw. dadurch erreicht werden, dass das exzentrisch angeordnete erste Arbeitslumen mit den anderen Arbeitslumen verbunden, insbesondere stoffschlüssig verbunden ist.

Der Katheter kann auch mit anderen Systemen verwendet werden.

Die Erfindung wird nachfolgend anhand von Ausführungsbeispielen mit weiteren Einzelheiten unter Bezugnahme auf die beigefügten schematischen Zeichnungen näher erläutert. In diesen zeigen
- Fig. 1: eine Ansicht eines Systems nach einem erfindungsgemäßen Ausführungsbeispiel in einem Blutgefäß mit expandierter Rekanalisationseinrichtung, wobei für die Positionierung der Rekanalisationseinrichtung eine zusätzliche Zuführleitung in einem Kanal vorgesehen ist;
- Fig. 2: eine Ansicht eines Systems nach einem weiteren erfindungsgemäßen Ausführungsbeispiel in einem Blutgefäß mit expandierter Rekanalisationseinrichtung, wobei die Rekanalisationseinrichtung ohne zusätzliche Zuführleitung direkt durch den Kanal positionierbar ist;
- Fig. 3: einen Längschnitt durch das distale Ende eines Katheters mit drei konzentrischen Arbeitslumen, der für die Systeme gemäß Fig. 1, 2 geeignet ist;
- Fig. 4: einen Längschnitt durch den Katheter gemäß Fig. 3 mit einer Zuführleitung im Kanal entsprechend dem System gemäß Fig. 1;
- Fig. 5: einen Längschnitt durch den Katheter gemäß Fig. 3 mit einer im Kanal unmittelbar zugeführten Rekanalisationseinrichtung entsprechend dem System gemäß Fig. 2;
- Fig. 6: einen Längschnitt durch das distale Ende eines Katheters mit zwei konzentrischen Arbeitslumen, der für die Systeme gemäß Fig. 1, 2 geeignet ist;
- Fig. 7: eine Ansicht eines Beispiels für einen Katheter mit drei Arbeitslumen, von denen eines exzentrisch angeordnet ist und der für die Systeme gemäß Fig. 1, 2 geeignet ist;
- Fig. 8: eine perspektivische Ansicht des distalen Endes des Katheter gemäß Fig. 7
- Fig. 9: einen Schnitt durch den Katheter gemäß Fig. 7;
- Fig. 10: einen Schnitt durch einen weiteren Katheter, der für die Systeme gemäß Fig. 1, 2 geeignet ist und
- Fig.11: einen Längsschnitt durch den Katheter gemäß Fig. 10.

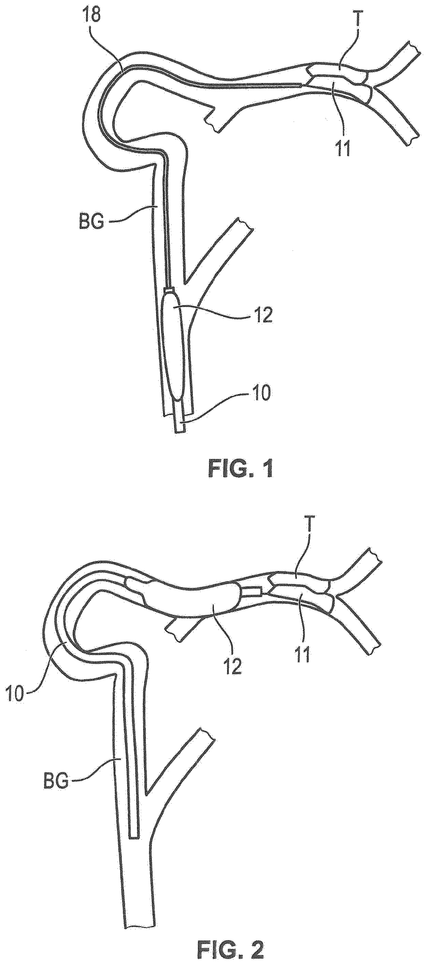

In den Fig. 1 und 2 sind medizinische Systeme nach erfindungsgemäßen Beispielen im Gebrauch dargestellt, die in ein Blutgefäß, bspw. die Halsschlagader und die mittlere Zerebralarterie (MCA) eingeführt sind. Die Systeme können an anderen Behandlungsstellen im Körper eingesetzt werden.

Den beiden Systemen ist gemeinsam, dass diese Komponenten zum endovaskulären Temperieren von Blut, insbesondere für die endovaskuläre Hypothermie, und Komponenten zur Rekanalisation eines Blutgefäßes in ein- und demselben System kombinieren. Das System ist einheitlich handhabbar, so dass ohne einen Gerätewechsel sowohl die Kühl- als auch Rekanalisationsfunktion durch die Komponenten des Systems bei der Behandlung erfüllt werden können. Dabei können die Blutkühlung und die Rekanalisation zeitlich unabhängig oder zeitgleich voneinander erfolgen. Beide Systeme ermöglichen die Blutkühlung vor, während und nach der Rekanalisation. Es ist auch möglich, in Abhängigkeit von der jeweils gewählten Rekanalisationsfunktion die Kühlung nur vor oder nach der Rekanalisation durchzuführen, beispielsweise wenn während des Rekanalisationsvorgangs keine Durchströmung des zu behandelnden Gefäßes möglich ist.

Die Erfindung wird anhand der kombinierten Temperier und Rekanalisationsfunktionen beschrieben. Die damit offanbarten Merkmale und Vorteile werden auch im Zusammenhang mit einem System offanbart und beansprucht, das allgemein eine Temperiereinrichtung mit einer Behandlungseinrichtung, wie im Anspruch 1 angegeben, kombiniert.

Für die kombinierte Kühlung und Rekanalisation weisen die Systeme gemäß Fig. 1 und 2 eine Zuführung 10, insbesondere einen Katheter, eine Rekanalisationseinrichtung 11 und ein Temperierelement 12 auf. Die Rekanalisationseinrichtung 11 und das Temperierelement 12 bilden gesonderte Komponenten des Systems, die räumlich und funktionell voneinander getrennt sind.

Die Rekanalisationseinrichtung 11 ist radial komprimierbar, so dass diese auf der proximalen Anwenderseite des Systems in die Zuführung 10 eingeführt und in der Zuführung 10 im komprimierten Zustand axial beweglich ist. Damit kann die Rekanalisationseinrichtung 11 durch die Zuführung 10 an den Behandlungsort bspw. mittels eines Transportdrahtes 23 oder eines Pushers, der die Rekanalisationseinrichtung 11 bewegt, transportiert werden.

Die Rekanalisationseinrichtung 11 ist radial expandierbar, wenn diese aus der Zuführung 10 am Behandlungsort, d.h. in den Fig. 1 und 2 im Bereich des Thrombus, entlassen wird. Bei der radialen Expansion vergrößert sich der Durchmesser der Rekanalisationseinrichtung 11, so dass der Thrombus an die Gefäßwand gedrückt und eine Öffnung im Blutgefäß gebildet wird. Die radiale Expandierbarkeit kann ohne äußere Krafteinwirkung erfolgen. Die Rekanalisationseinrichtung 11 ist in diesem Fall selbstexpandierbar, beispielsweise aufgrund von Federkräften, die im komprimierten Zustand gespeichert sind, oder durch die Wahl eines Formgedächtniswerkstoffes, der entsprechend konditioniert ist. Beide Mechanismen sind an sich bekannt. Es ist auch möglich, die Rekanalisationseinrichtung 11 durch Aufbringen äußerer Radialkräfte zu expandieren, beispielsweise durch einen Dilatationskatheter.

Die Rekanalisationseinrichtung 11 kann eine röhrchenförmige oder stentartige Gitterstruktur aufweisen, die beispielsweise geflochten oder lasergeschnitten ist. Die Expansionsmechanismen derartiger Gitterstrukturen sind bekannt.

Die vorgenannten Merkmale der Rekanalisationseinrichtung 11 werden im Zusammenhang mit allen Beispielen offenbart.

Das System gemäß Fig. 1 und 2 weist ferner ein Temperierelement 12 auf, das zum Temperieren von Blut vorgesehen ist. Zusammen mit dem System wird auch das Temperiermittel bzw. Temperiermedium, insbesondere das Kühlmittel, offenbart und beansprucht, das Teil des Systems ist.

Das Temperierelement 12 ist in der Form eines Ballons 19 ausgebildet. Der Ballon 19 ist an der Außenseite der Zuführung 10, insbesondere an der Außenwandung des Katheters, angebracht. Die Zuführung 10 geht durch das Temperierelement 12 hindurch. Das Temperierelement 12 umgibt zumindest teilweise, insbesondere vollständig, die Zuführung 10 in Umfangsrichtung und erstreckt sich in Längsrichtung entlang der Zuführung 10, insbesondere eines Abschnittes der Zuführung 10.

Das Temperierelement 12, insbesondere der Ballon 19, ist radial expandierbar. Im expandierten Zustand ist der Außendurchmesser des Ballons 19 größer als der Außendurchmesser der Temperierleitung 13. Außerdem ist der Ballon 19 so dimensioniert, dass dieser im Zielgefäß einen kleineren Außendurchmesser als der Gefäßdurchmesser aufweist (s. Fig. 1). Dazu ist der Ballon 19 auf einen vorgegebenen Durchmesser expandierbar (keine Compliance). Im Gebrauch strömt das Blut durch den Spalt zwischen dem Ballon 19 und der Gefäßwand. Der Ballon 19 kann hierfür unprofiliert sein, d.h. er weist eine glatte, kontinuierliche Wandung auf.

Zur Verbesserung der Durchströmbarkeit kann der Ballon 19 im expandierten Zustand eine profilierte Außenwand bilden, so dass das Blut in distaler Richtung am Ballon 19 vorbeiströmen kann. Dabei können erste Wandsegmente des Ballons 19 an der Gefäßwand anliegen. Dazwischen angeordnete zweite Wandsegmente können einen kleineren Außendurchmesser als die ersten Wandsegmente aufweisen und Strömungskanäle für das Blut bilden. Im Querschnitt kann der Ballon 19 bspw. sternförmig ausgebildet sein.

Beim Vorbeiströmen gibt das Blut Wärme an den Ballon 19 ab, der somit als Wärmetauscher fungiert.

Das so temperierte bzw. gekühlte Blut gelangt dann in den Bereich der Rekanalisationseinrichtung 11 und, sofern diese durchlässig ist, durchströmt diese während des Rekanalisationsvorganges. Andere Betriebsarten sind möglich, wie eingangs beschrieben.

Das System ist dazu angepasst, dass die Rekanalisationseinrichtung 11 im Gebrauch distal zum Temperierelement 12 positioniert werden kann. Damit wird erreicht, dass im Gebrauch durch das Temperierelement 12 temperiertes Blut zur Rekanalisationsstelle strömen kann. Bei den Beispielen gemäß Fig. 1 und 2 wird dies dadurch erreicht, dass die Rekanalisationseinrichtung 11 in der Zuführung 10 im komprimierten Zustand angeordnet ist und in Längsrichtung der Zuführung 10 bewegt werden kann. Das Temperierelement 12 ist mit der Zuführung 10 verbunden, konkret mit einer Außenwandung der Zuführung 12. Das distale Ende des Temperierelements 12 endet in etwa auf derselben Höhe wie das distale Ende der Zuführung 10. Das distale Ende des Temperierelements 12 kann in proximaler Richtung vom distalen Ende der Zuführung 10 beabstandet sein. Dadurch wird eine Kollision des Temperierelements 12 mit anderen Elementen, wie beispielsweise dem Rekanalisationselement 11sicher vermieden, die aus der am distalen Ende der Zuführung 10 vorgesehenen Austrittsöffnung austreten. Mit anderen Worten erstreckt sich das Temperierelement 12 ausgehend vom distalen Ende der Zuführung 10 in proximaler Richtung entlang der Zuführung 10.

Wenn die Rekanalisationseinrichtung 11 aus der Zuführung 10 entlassen wird, sei es direkt, wie in Fig. 2 oder indirekt, wie in Fig. 1, befindet sich die Rekanalisationseinrichtung 11 distal zum Temperierelement 12. Damit wird die in den Fig. 1 und 2 gezeigte sequenzielle Anordnung des Temperierelements 12 und der Rekanalisationseinrichtung 11 erreicht, so dass durch das Temperierelemet 12 temperiertes Blut zur Rekanalisationsstelle gelangen kann.

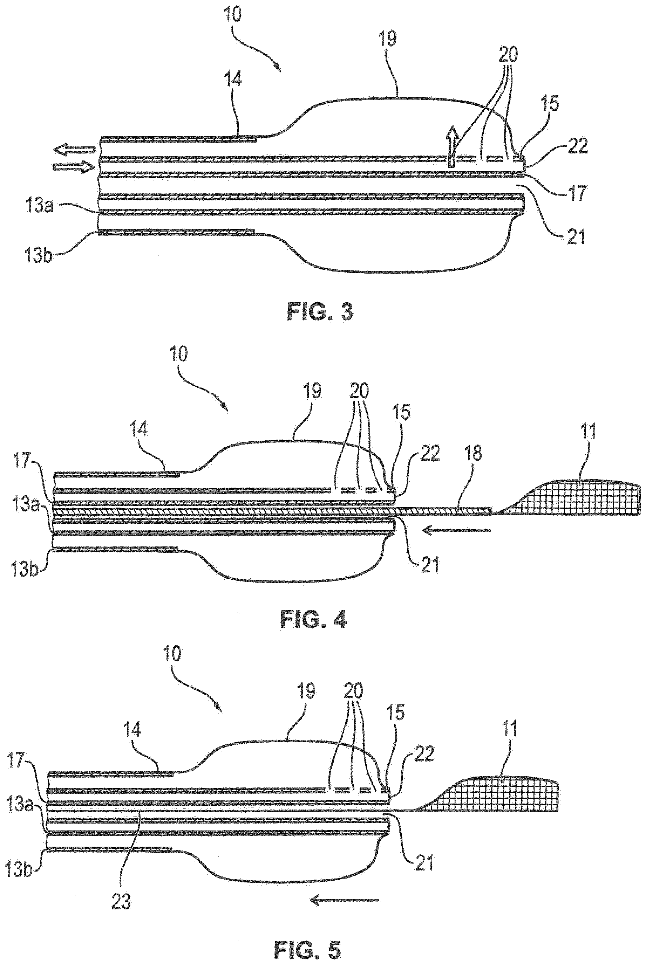

Die Zuführung 10 ist so aufgebaut, dass diese sowohl die Rekanalisationseinrichtung 11 an dem Behandlungsort transportieren als auch das Temperierelement 12 mit einem Temperiermedium versorgen kann. Die Zuführung 10, konkret ein Katheter, weist dazu einen Kanal 17 für die Rekanalisationseinrichtung 11 auf. Der Kanal 17 wird im Zusammenhang mit den Fig. 3 bis 6 näher beschrieben. Der Kanal 17 erstreckt sich entlang wenigstens einer Temperierleitung 13a, 13b, die von dem Temperiermedium im Gebrauch durchströmt wird und die mit dem Temperierelement 12 fluidverbunden ist. Die Zuführung 10 integriert den Kanal 17 und die wenigstens eine Temperierleitung 13a, 13b in ein einziges Bauteil, insbesondere in eine Katheterleitung bzw. Hauptleitung 21, so dass der Kanal 17 und die wenigstens eine Temperierleitung 13a, 13b zusammen gehandhabt werden können bzw. handhabbar sind. Mit dem Begriff "Kanal" wird sowohl das Lumen als auch die Wandung bezeichnet, die das Lumen begrenzt. Dasselbe gilt für den Begriff "Temperierleitung".

Nachfolgend werden zwei Möglichkeiten beschrieben, wie die Doppelfunktion der Zuführung 10, nämlich die Zuführung der Rekanalisationseinrichtung 11 und die Versorgung des Temperierelements 12, verwirklicht werden kann. Im Beispiel gemäß Fig. 1 weist die Zuführung 10 eine gesonderte Zuführleitung 18 auf, die im Kanal 17 längsbeweglich angeordnet ist. Die Rekanalisationseinrichtung 11 wiederum ist in der Zuführleitung 18 längsbeweglich angeordnet. Die Zuführleitung 18 kann beispielsweise als so genannter Mikrokatheter ausgebildet sein, also ein flexibler Katheter mit einem kleinen Außendurchmesser, der in englumige Gefäßbereiche vordringen kann. Wie in Fig. 1 gezeigt, hat dies den Vorteil, dass das Temperierelement 12 mit relativ großem Abstand vom Thrombus T in einem Gefäß mit vergleichweise großem Durchmesser positioniert werden kann. Die Zuführleitung 10 wird, beispielsweise mittels eines Führungsdrahtes, aus dem Kanal 17 vorgeschoben und verlässt die Zuführung 10. Die Zuführleitung 18 wird bis zur Behandlungsstelle bewegt, und zwar soweit, bis die Spitze bzw. das distale Ende der Zuführleitung 18 über den Thrombus T in distaler Richtung vorsteht. Die Zuführleitung 18 bildet somit eine teleskopierbare Verbindung, insbesondere mit zwei Schüblingen, zwischen dem mit der Zuführung 10 verbundenen Temperierelement 12 und dem Thrombus T. Die Rekanalisationseinrichtung 11 kann dann bis zum Thrombus T durch die Zuführleitung 18 vorgeschoben werden, die, wenn die Rekanalisationseinrichtung 11 korrekt im Bereich des Thrombus positioniert ist, in an sich bekannter Weise zurückgezogen wird. Dadurch wird die Rekanalisationseinrichtung 11, wie in Fig. 1 dargestellt, aus der Zuführleitung 18 entlassen und kann radial expandieren.

Damit ist die Rekanalisationseinrichtung 11 distal vom Temperierelement 12 positioniert, bzw. durch das System positionierbar.

Alternativ kann die Rekanalisationseinrichtung 11 direkt im Kanal 17 längsbeweglich angeordnet sein, wie in Fig. 2 dargestellt. Damit wird der Aufbau des Systems vereinfacht. Da die Rekanalisationseinrichtung 11 unmittelbar nach dem Freisetzen aus dem Kanal 17 expandiert, ist der Abstand zwischen dem Temperierelement 12 und der freigesetzten Rekanalisationseinrichtung 11 kleiner als der mit dem System gemäß Fig. 1 einstellbare Abstand.

Bei dem Ausführungsbeispiel gemäß Fig. 2 kann der Abstand zwischen dem Temperierelement 12 und der Rekanalisationseinrichtung 11 dadurch eingestellt werden, dass das Temperierelement 12 nach dem Entlassen der Rekanalisationseinrichtung 11 weiter in proximale Richtung zurückgezogen wird und zwar zusammen mit der Zuführung 10 bzw. dem Katheter. Beim Zurückziehen ist das Temperierelement 12 komprimiert bzw. befindet sich nicht im expandierten Zustand. Im Unterschied dazu kann das Temperierelement 12 des Beispiels gemäß Fig. 1 an einer geeigneten Stelle des Blutgefäßes ortsfest positioniert werden. Der Abstand zwischen dem Temperierelement 12 und der Rekanalisationseinrichtung 11 wird durch das Vorschieben der Zuführleitung 18 eingestellt. Es ist ersichtlich, dass hierbei nahezu beliebig große Abstände möglich sind. Das System gemäß Fig. 1 ist also besondere gut dafür geeignet, Verschlüsse in Blutgefäßen mit sehr kleinem Durchmesser zu rekanalisieren und das zur Rekanalisationsstelle strömende Blut wirkungsvoll zu kühlen.

Die Positionierbarkeit der Rekanalisationseinrichtung 11 distal zum Temperierelement 12 wird also dadurch erreicht, dass die Rekanalisationseinrichtung 11 und das Temperierelement 12 zum Positionieren der Rekanalisationseinrichtung 11 relativ zueinander axialbeweglich sind. Die Relativbewegung kann beispielsweise dadurch erreicht werden, dass das Temperierelement 12 bezogen auf das Gesamtsystem ortsfest angeordnet ist und die Rekanalisationseinrichtung 11 in der Zuführung 10 direkt oder indirekt durch die Zuführleitung 18 axial bewegt wird. Die Relativbeweglichkeit ermöglicht die Positionierbarkeit der Rekanalisationseinrichtung distal zum Temperierelement 12, da die Rekanalisationseinrichtung 11 über die Position des Temperierelements 12 von proximal nach distal transportiert und distal zum Temperierelement 12 aus der Zuführung 10 entlassen werden kann. Die Relativbeweglichkeit zwischen der Rekanalisationseinrichtung 11 und dem Temperierelement 12 ermöglicht ferner die Einstellung des axialen Abstandes zwischen dem Temperierelement 12 und der Rekanalisationseinrichtung 11, wodurch patientenspezifischen Gegebenheiten wie Gefäßdurchmesser, Gefäßkrümmung usw. berücksichtigt werden können.

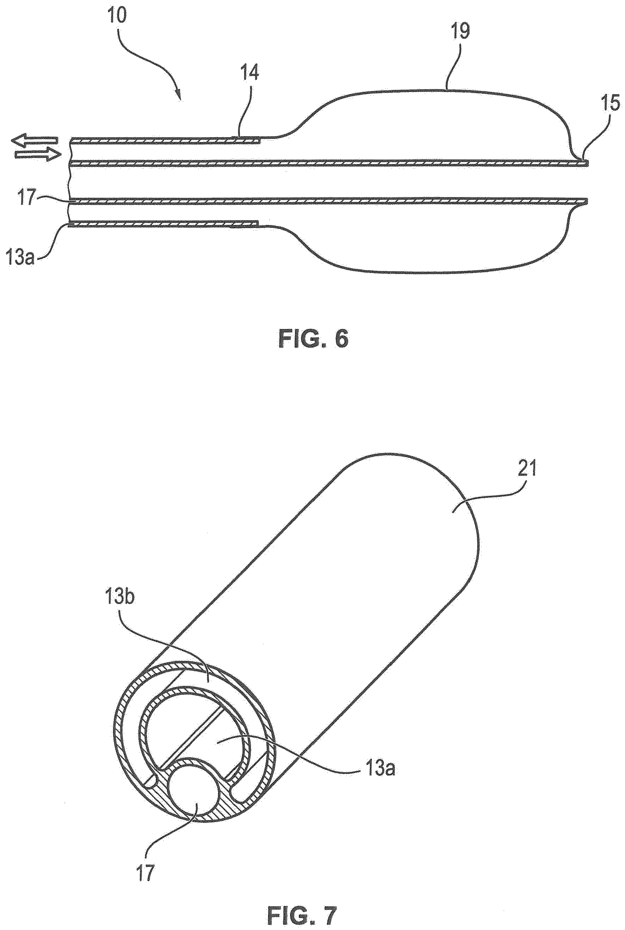

Beispiele für die Verbindung des Temperierelements 12 mit der Zuführung 10 sind in den Fig. 3 bis 6 dargestellt, wobei die Fig. 3 bis 5 eine dreilumige Zuführung 10 und die Fig. 6 eine zweilumige Zuführung 10 darstellen.

Beiden Zuführungen 10 gemäß Fig. 3 und 6 ist der Kanal 17 gemeinsam, der für die direkte Zuführung des Rekanalisationselements 11 (Fig. 5) oder für die indirekte Zuführung durch die zusätzliche Zuführleitung 18 (Fig. 4) angepasst ist. Die beiden Zufuhrmöglichkeiten gemäß Fig. 4, 5 sind auch bei der Zuführung 10 gemäß Fig. 6 möglich.

Der Kanal 17 bildet eine flexible Leitung bzw. einen flexiblen Schlauch. Der Außendurchmesser der Rekanalisationseinrichtung 11 (Fig. 5) bzw. der Außendurchmesser der zusätzlichen Zufuhrleitung 18 (Fig. 4) ist auf den Innendurchmesser des Kanals 17 abgestimmt, so dass die Rekanalisationseinrichtung 11 bzw. die Zuführleitung 18 im Kanal 17 axial bewegt werden kann. Dasselbe gilt für den Kanal 17 gemäß Fig. 6.

Die Zuführung 10 gemäß Fig. 3 weist wenigstens eine weitere, wenigstens zwei Temperierleitungen 13a, 13b auf, die sich entlang des Kanals 17 erstrecken. Konkret sind bei dem Beispiel gemäß Fig. 3 der Kanal 17 und die beiden Temperierleitungen 13a, 13b im Wesentlichen konzentrisch angeordnet. Andere Querschnittsgeometrien der Zuführung 10 sind möglich, wie anhand des Ausführungsbeispiels gemäß Fig. 7 näher erläutert.

Bei dem Beispiel gemäß Fig. 3 sind die beiden Temperierleitungen 13a, 13b für eine kontinuierliche Durchströmung des Temperierelements 12, konkret des Ballons 19 angepasst. Dazu dient eine der beiden Temperierleitungen 13a, insbesondere die erste Temperierleitung 13a als Vorlaufleitung, durch die das Temperiermedium bzw. Kühlmedium dem Ballon 19 zugeführt wird. Die andere der beiden Temperierleitungen, insbesondere die zweite Temperierleitung 13b dient als Rücklaufleitung, durch die das Temperiermedium aus dem Ballon 19 abgeführt wird.

Die erste und zweite Temperierleitung 13a, 13b sind durch flexible Leitungen bzw. Schläuche mit unterschiedlichen Durchmessern gebildet. Die Innenwandung der ersten Temperierleitung 13a für den Vorlauf ist durch die Außenwandung des Kanals 17 gebildet. Damit ergibt sich ein erster Ringspalt zwischen dem Kanal 17 und der Innenwandung der ersten Temperierleitung 13a, durch die das Temperiermedium in distaler Richtung zugeführt wird. Die Außenwandung der ersten Temperierleitung 13a bildet zusammen mit der Innenwandung der zweiten Temperierleitung 13b einen zweiten Ringspalt, durch den das erwärmte Temperiermedium aus dem Ballon 19 abgeführt wird. Die Zufuhrrichtungen des Temperiermediums sind durch die beiden Pfeile in Fig. 3 (siehe auch Fig. 6) kenntlich gemacht.

Die erste Temperierleitung 13a befindet sich bei dem Beispiel gemäß Fig. 3 zwischen dem Kanal 17 und der zweiten Temperierleitung 13b. Die zweite Temperierleitung 13b bildet die Außenwandung der Zuführung 10, die im Gebrauch mit dem Blut in Kontakt kommt.

Die erste Temperierleitung 13a für den Vorlauf ist innerhalb der zweiten Temperierleitung 13b für den Rücklauf angeordnet. Damit befindet sich die zweite Temperierleitung 13b zwischen dem umgebenden Blut und der ersten Temperierleitung, so dass das zugeführte Temperiermedium durch das rückgeführte Temperiermedium gegen das Blut thermisch isoliert wird.

Dasselbe Isolierungsprinzip liegt der Zuführung 10 gemäß Fig. 7 zu Grunde.

Das Temperierelement 12, konkret der Ballon 19 ist mit der Zuführung 10 wie folgt fluidverbunden.

Die erste Temperierleitung 13a für den Vorlauf steht in distaler Richtung über das distale Ende der zweiten Temperierleitung 13b für den Rücklauf vor. Die erste Temperierleitung 13a ist also - zumindest am distalen Ende der Zuführung 10 - länger als die zweite Temperierleitung 13b für den Rücklauf. Das distale Ende 15 des Ballons 19 ist mit dem vorstehenden Abschnitt der ersten Temperierleitung 13a für den Vorlauf verbunden, wie in Fig. 3 gut zu erkennen. Konkret ist das distale Ende 15 des Temperierelements 12 bzw. des Ballons 19 mit dem distalen Ende der ersten Temperierleitung 13a verbunden.

Das distale Ende der ersten Temperierleitung 13a weist ein Dichtelement 22 zwischen der ersten Temperierleitung 13a und dem Kanal 17 auf, wodurch verhindert wird, dass das Temperiermedium aus dem Blutgefäß strömen kann. Mit anderen Worten ist der Ringspalt zwischen dem Kanal 17 und der ersten Temperierleitung 13a distal vom Temperierelement 12 bzw. dem Ballon 19 fluiddicht verschlossen.

Das proximale Ende 14 des Temperierelements 12 bzw. des Ballons 19 ist mit der zweiten Temperierleitung 13b fluiddicht verbunden. Konkret ist das proximale Ende 14 des Ballons 19 mit dem distalen Ende der zweiten Temperierleitung 13b fluiddicht verbunden.

Die Fluidverbindung zwischen der ersten Temperierleitung 13a und dem Ballon 19 erfolgt durch wenigstens eine Austrittsöffnung 20, beispielsweise durch 2, 3, oder mehr Austrittsöffnungen 20, die seitlich in der Wandung der ersten Temperierleitung 13a vorgesehen sind. Die Anzahl und Anordnung der Austrittsöffnungen 20 sowohl in Längs- als auch in Umfangsrichtung der ersten Temperierleitung 13a kann von dem Beispiel gemäß Fig. 3 abweichen.

Der Austritt des Temperiermediums aus dem Ballon 19 erfolgt durch den Ringspalt zwischen der zweiten Temperierleitung 13b und der ersten Temperierleitung 13a. Damit sind die erste und zweite Temperierleitung 13a, 13b am distalen Ende miteinander fluidverbunden, so dass das Temperiermedium durch den Ballon 19 kontinuierlich strömen kann. Der Ballon 19 überspannt den Abstand zwischen dem distalen Ende der ersten Temperierleitung 13a und dem distalen Ende der zweiten Temperierleitung 13b und somit den vorstehenden Abschnitt der ersten Temperierleitung 13a. Damit ergibt sich ein Aufnahmeraum für das Temperiermedium, der fluiddicht mit der Zuführung 10 verbunden ist.

Für eine effektive Wirkung des Ballons 19 als Wärmetauscher sind die Austrittsöffnungen 20 im Bereich des distalen Endes der ersten Temperierleitung 13a vorgesehen, so dass das kühle Temperiermedium entlang der vorstehenden ersten Temperierleitung 13a im Ballon 19 in proximaler Richtung zurückströmen kann. Im Bereich des Ballons 19 erfolgt der Wärmeübergang vom umgebenden wärmeren Blut zum kühlen Temperiermedium im Ballon 19.

Durch die Länge des vorstehenden Abschnittes der ersten Temperierleitung 13a kann die Länge der Kühlstrecke eingestellt werden.

Fig. 4, 5 zeigen die unterschiedlichen Einsatzmöglichkeiten der Zuführung 10 bzw. des Katheters gemäß Fig. 3. Das Beispiel gemäß Fig. 4 weist die zusätzliche Zuführleitung 18 auf, mit der die Zuführung 10 für die Zufuhr der Rekanalisationseinrichtung 11 teleskopierbar ist. Die Rekanalisationseinrichtung 11 ist mit einem Transportdraht verbunden, der in der Zuführleitung 18 geführt ist, so dass die Rekanalisationsrichtung 11 in der Zuführleitung 18 axial beweglich angeordnet ist. Die Rekanalisationseinrichtung 11 ist in die Zuführleitung 18 nach dem Entlassen wieder einziehbar. Ein Beispiel für ein wiedereinziehbares Geflecht ist in der auf die Anmelderin zurückgehenden Anmeldung 10 2009 056 450 veröffentlicht.

Bei dem Beispiel gemäß Fig. 5 ist die Rekanalisationseinrichtung 11 direkt im Kanal 17 geführt. Das bedeutet, dass die Innenwandung des Kanals 17 die Rekanalisationseinrichtung 11 im komprimierten Zustand hält, die nach dem Entlassen aus dem Kanal 17 radial expandiert, wie in Fig. 5 dargestellt. Auch bei diesem Beispiel ist die Wiedereinziehbarkeit der Rekanalisationseinrichtung 11 gegeben, wie durch den Pfeil in proximaler Richtung dargestellt. Hierzu wird der Transportdraht 23 entsprechend betätigt.

Die Zuführung 10 gemäß Fig. 6 unterscheidet sich von der Zuführung 10 gemäß Fig. 3 darin, dass eine einzige Temperierleitung 13a sowohl für die Zufuhr als auch für die Entsorgung des Temperiermittels vorgesehen ist, wie durch den Doppelpfeil in Fig. 6 verdeutlicht. Ähnlich wie bei dem Beispiel gemäß Fig. 3 umgibt die einzige Temperierleitung 13a den Kanal 17, wobei der Kanal 17 und die Temperierleitung 13a jeweils als flexible Schläuche bzw. Leitungen ausgebildet sind. Konkret sind der Kanal 17 und die Temperierleitung 13a konzentrisch angeordnet.

Das Beispiel gemäß Fig. 6 ist für den pulsatilen Betrieb geeignet, wobei der Ballon 19 abwechselnd mit Temperiermedium gefüllt und entleert wird. Dazu ist das distale Ende 15 des Temperierelements 12, konkret des Ballons 19 mit dem Abschnitt des Kanals 17 verbunden, der über das distale Ende der einzigen Temperierleitung 13a in distaler Richtung vorsteht. Konkret ist das distale Ende 15 des Ballons 19 mit dem distalen Ende des Kanals 17 fluiddicht verbunden. Das proximale Ende der Temperiereinrichtung 12, konkret des Ballons 19, ist mit der Temperierleitung 13a, konkret mit dem distalen Ende der Temperierleitung 13a verbunden. Dadurch wird, ähnlich wie bei dem Beispiel gemäß Fig. 3, eine Kühlstrecke gebildet, die in etwa der Länge des vorstehenden Abschnitts des Kanals 17 entspricht. Der vorstehende Abschnitt des Kanals 17 wird, wie beim Beispiel gemäß Fig. 3 durch die Ballonwandung 19 überspannt, wodurch ein Aufnahmeraum für das Temperiermedium gebildet ist. Insoweit wird auch auf die Beschreibung der Fig. 3 Bezug genommen.

Für die Versorgung des Temperierelements 12 mit Temperiermedium ist die wenigstens eine Temperierleitung, bzw. sind beide Temperierleitungen 13a, 13b mit einer nicht dargestellten Versorgungseinheit verbunden, die extrakorporal angeordnet ist und die einen ausreichenden Versorgungsdruck stellt.

Die Zuführung 10 bildet einen multifunktionalen Katheter mit Rekanalisations- und Temperierfunktion, insbesondere Kühlfunktion.

Für die Aufnahme der Zuführleitung 18 hat das Lumen des Kanals 17 einen Durchmesser von mindestens 0,6 mm, insbesondere von mindestens 0,7 mm, insbesondere von mindestens 0,8 mm, insbesondere von mindestens 0,9 mm, insbesondere von 1,0 mm, insbesondere von 1,1 mm, insbesondere von 1,2 mm, insbesondere von 1,4 mm. Dadurch wird eine im Wesentlichen reibungslose Axialverschieblichkeit der Zuführleitung 18, insbesondere des Mikrokatheters erreicht. Der maximale Innendurchmesser des Kanals 17 beträgt 1,6 mm, insbesondere 1,4 mm, insbesondere 1,2 mm, insbesondere 1,0 mm, insbesondere 0,8 mm. Die vorgenannten Obergrenzen werden jeweils mit den vorstehend genannten Untergrenzen zur Bildung von Bereichen offenbart, also die Obergrenze 1,6 mm mit allen Untergrenzwerten, die Obergrenze von 1,4 mm mit allen Untergrenzwerten usw. Die Obergrenzwerte bewirken, dass sich die Zuführleitung 18 im Kanal 17 nicht wesentlich wellt, wenn unterschiedliche, an den Kanal 17 entsprechend angepasste Zuführleitungen 18 verwendet werden.

Das Lumen des Kanals 17 gemäß Fig. 2 und 5, bei dem die Rekanalisationseinrichtung 11 unmittelbar im Kanal 17 geführt ist, weist einen minimalen Durchmesser von 0,35 mm, insbesondere von 0,40 mm, insbesondere von 0,45 mm, insbesondere von 0,5 mm, insbesondere von 0,6 mm, insbesondere von 0,7 mm auf. Damit wird die Zufuhr der Rekanalisationseinrichtung 11 ohne wesentliche Reibung erreicht. Der maximale Innendurchmesser des Kanals 17 beträgt 1,0 mm, insbesondere 0,9 mm, insbesondere 0,8 mm, insbesondere 0,7 mm, insbesondere 0,6 mm insbesondere 0,5 mm. Die vorstehend genannten Obergrenzen werden jeweils einzeln mit den vorstehend genannten Untergrenzen zusammen offenbart, d.h. die Obergrenze von 1,0 mm wird mit allen Untergrenzwerten zusammen offenbart, die Obergrenze von 0,9 mm wird zusammen mit allen Untergrenzwerten offenbart usw. Die Einstellung des maximalen Innendurchmessers bewirkt, dass sich der Transport bzw. Führungsdraht der Vorrichtung im Lumen nicht wesentlich wellt.

Die einzelnen Komponenten des Systems, d.h. die Zuführung 10, die Rekanalisationseinrichtung 11 und das Temperierelement 12 bilden wesentliche Elemente der Erfindung. Es ist möglich, beispielsweise die Zuführleitung 18 und die Rekanalisationseinrichtung 11 einerseits und die Zuführung 10 mit dem Temperierelement 12 andererseits separat anzubieten, wobei die Kombination dieser Komponenten zu dem medizinischen System anderweitig erfolgt.

Ein Beispiel eines Katheters ist in den Fig. 7 bis 9 dargestellt. Der dort gezeigte Katheter wird im Zusammenhang mit dem vorstehend beschriebenen und beanspruchten medizinischen System zur Temperierung und Rekanalisation offenbart. Es ist möglich, den Katheter auch für andere Behandlungen mit anderen Systemen zu verwenden.

Der Katheter gemäß Fig. 7 bildet eine Hauptleitung 21, die die Außenummantelung des Katheters darstellt. In der Hauptleitung 21 sind wenigstens drei Arbeitslumen 13a, 13b, 17 ausgebildet. Mehr als drei Arbeitslumen sind möglich. Bei dem Beispiel gemäß Fig. 7 sind genau drei Arbeitslumen 13a, 13b, 17 vorgesehen. Das erste Arbeitslumen 17 ist exzentrisch bezogen auf die Mittellinie der Hauptleitung 21 angeordnet. Die Hauptleitung 21 ist röhrchenförmig und weist eine (nicht dargestellte) Mittellinie im Zentrum auf. Bezogen auf diese Mittellinie ist das erste Arbeitslumen 17 exzentrisch angeordnet, wie in Fig. 7 zu sehen. Mit anderen Worten ist das Arbeitslumen 17 seitlich, d.h. radial versetzt an der Wandung der Hauptleitung 21 ausgebildet. Das Arbeitslumen 17 weist einen im Wesentlichen kreisförmigen Querschnitt auf.

Das Arbeitslumen 17 entspricht dem Kanal 17 in den Beispielen gemäß Fig. 1 bis 6. Im Gebrauch wird im Kanal 17 bzw. im Arbeitslumen 17 die Rekanalisationseinrichtung 11 bzw. die Zuführleitung 18 angeordnet und bewegt.

Die Hauptleitung 21 weist ein zweites und drittes Arbeitslumen 13a und 13b auf. Wie in Fig. 7 zu sehen, sind das zweite und dritte Arbeitslumen 13a, 13b im Wesentlichen konzentrisch zueinander angeordnet. Das zweite und dritte Arbeitslumen 13a, 13b können konzentrisch zur Hauptleitung 21 ausgebildet sein, bzw. konzentrisch zueinander, wenn die Hauptleitung 21 dem dritten Arbeitslumen 13b entspricht.

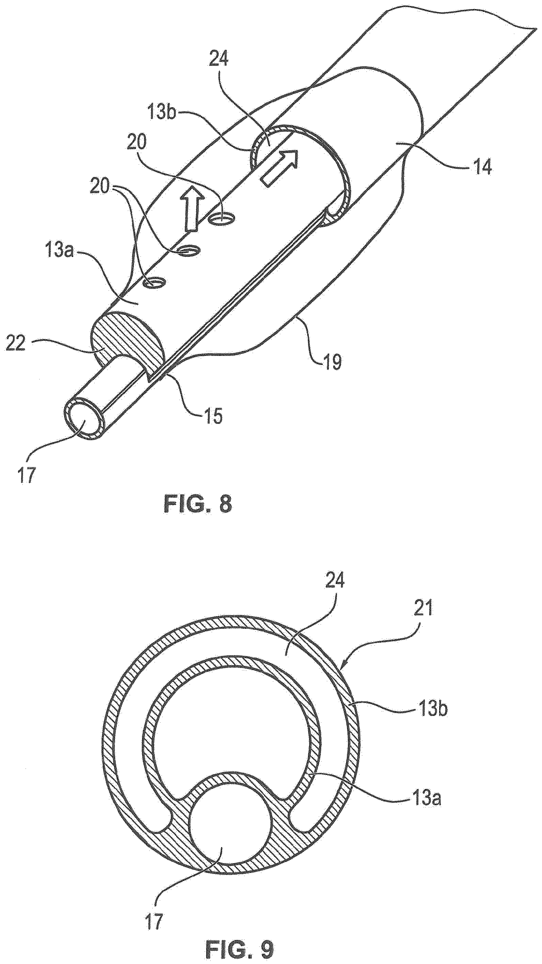

Konkret weist der Querschnitt des zweiten Arbeitslumens 13a und des dritten Arbeitslumens 13b im Wesentlichen denselben Mittelpunkt auf. Im Querschnitt sind das zweite und dritte Arbeitslumen 13a, 13b im Wesentlichen kreisförmig, wie in Fig. 9 gut zu erkennen. Die Kreisgeometrie des zweiten und dritten Arbeitslumens 13a, 13b wird durch das erste Arbeitslumen 17 in Umfangsrichtung unterbrochen, wie in Fig. 9 zu erkennen. Das zweite und dritte Arbeitslumen 13a, 13b sind jeweils mit dem ersten Arbeitslumen verbunden, insbesondere stoffschlüssig verbunden, wodurch sich eine positionsfeste Anordnung der Lumen untereinander ergibt.

Das zweite und dritte Arbeitslumen 13a, 13b entsprechen der ersten und zweiten Temperierleitung 13a, 13b der Beispiele gemäß Fig. 1 und 6. Das zweite Arbeitslumen 13a bzw. die erste Temperierleitung 13a dient der Zufuhr des Temperiermediums zum Temperierelement 12. Das dritte Arbeitslumen 13b bzw. die zweite Temperierleitung 13b dient dem Rücklauf des Temperiermediums aus dem Temperierelement 12. Wie in den vorangegangenen Beispielen ist zwischen der zweiten äußeren Temperierleitung 13b und der ersten inneren Temperierleitung 13a ein Ringspalt 24 ausgebildet. Der Ringspalt 24 ist durch die Wandung des exzentrisch angeordneten Kanals 17 unterbrochen. Im Unterschied zu den Ausführungsbeispielen gemäß Fig. 1 bis 6, bei denen alle Leitungen bzw. die Leitung und der Kanal konzentrisch angeordnet sind, wodurch sich ein vollständig umlaufender Ringspalt ergibt, ist bei dem Beispiel gemäß Fig. 9 der Ringspalt unterbrochen bzw. halbmondförmig oder C-förmig ausgebildet.

Der Vorteil des Katheters gemäß Fig. 7 bis 9 besteht darin, dass durch den exzentrisch angeordneten Kanal 17 die Querschnittsfläche der ersten Temperierleitung 13a vergrößert wird. Außerdem wird das Einlasslumen in der ersten Temperierleitung 13a durch das Auslasslumen der zweiten Temperierleitung 13b gegen das umgebende wärmere Blut thermisch isoliert, wodurch der Wärmeübergang zwischen der Kühlflüssigkeit bzw. allgemein dem Temperiermedium und dem Blut entlang des Katheters verringert wird.

Der erste Arbeitskanal 17 ist zum Zuführen eines Führungsdrahtes, oder, wie erwähnt, zum Zuführen eines Mikrokatheters geeignet. Das Lumen des ersten Arbeitslumens bzw. des Kanals 17 weist einen maximalen Durchmesser von 1,2 mm, insbesondere von 1,0 mm, insbesondere von 0,9 mm, insbesondere von maximal 0,85 mm, insbesondere von maximal 0,8 mm, insbesondere von maximal 0,75 mm auf. Die Untergrenze des Durchmessers beträgt minimal 0,4 mm, insbesondere minimal 0,5 mm, insbesondere minimal 0,6 mm, insbesondere minimal 0,7 mm, insbesondere minimal 0,8 mm. Die vorstehend genannten Untergrenzen können jeweils einzeln mit den vorstehend genannten Obergrenzen zur Bildung von Bereichen kombiniert werden, beispielsweise 0,4 mm mit allen Obergrenzwerten, 0,5 mm mit allen Obergrenzwerten usw. Die vorstehend genannten Ober- und Untergrenzwerte des Durchmessers gelten für den Kanal 17, wenn dieser zum Zuführen einer zusätzlichen Zuführleitung 18 bzw. eines Mikrokatheters ausgelegt ist.

Wenn das erste Arbeitslumen 17 bzw. der Kanal 17 zum Zuführen eines Führungsdrahtes vorgesehen ist, weist das erste Arbeitslumen 17 einen maximalen Durchmesser von höchstens 1,0 mm, insbesondere höchstens 0,8 mm, insbesondere höchstens 0,6 mm, insbesondere höchstens 0,5 mm, insbesondere höchstens 0,45 mm, insbesondere höchstens 0,4 mm, insbesondere höchstens 0,35 mm auf. Die Untergrenze des Durchmessers beträgt mindestens 0,3 mm, insbesondere mindestens 0,4 mm, insbesondere mindestens 0,5 mm, insbesondere mindestens 0,6 mm. Die vorstehend genannten Untergrenzwerte können jeweils mit allen Obergrenzwerten kombiniert werden, beispielsweise 0,3 mm mit allen Obergrenzwerten zum Zuführen des Führungsdrahtes, 0,4 mm mit allen Obergrenzwerten zum Zuführen des Zuführungsdrahtes usw.

Der Außendurchmesser des Katheters, konkret der Außendurchmesser der Hauptleitung 21, die im Beispiel gemäß Fig. 7 der Außenwandung der zweiten Temperierleitung 13b entspricht, kann folgende Dimensionen aufweisen. Der maximale Außendurchmesser kann 4,0 mm, insbesondere höchstens 3,5 mm, insbesondere höchstens 3,0 mm, insbesondere höchstens 2,7 mm, insbesondere höchstens 2,4 mm, insbesondere höchstens 2,0 mm, insbesondere höchstens 1,7 mm betragen. Besonders geeignet sind Katheter mit einer Größe von ca. 8 Fr (d.h. 2,7 mm) für die Zufuhr in die Halsschlagader geeignet.

Wenn der Katheter, wie im Beispiel gemäß Fig. 2 weiter distal zugeführt werden soll, ist ein Außendurchmesser von maximal 1,4 mm bis 1,0 mm möglich. Die Untergrenzen, die jeweils einzeln mit den vorgenannten Obergrenzen kombiniert werden können betragen mindestens 0,7 mm, insbesondere mindestens 1,0 mm, insbesondere mindestens 1,3 mm, insbesondere mindestens 1,7 mm.

Die Wandungen der beiden Temperierleitungen 13a, 13b sowie des Kanals 17 im Bereich der ersten Temperierleitung 13a können maximal 400 µm, insbesondere maximal 300 µm, insbesondere maximal 200 µm, insbesondere maximal 150 µm, insbesondere maximal 100 µm betragen. Die Untergrenze kann beispielsweise 90 µm betragen.

Die Verbindung des Temperierelements 12, konkret des Ballons 19 mit dem Katheter gemäß Fig. 7 ist in Fig. 8 dargestellt. Im Prinzip erfolgt die Verbindung ähnlich wie bei dem Ausführungsbeispiel gemäß Fig. 3. Insofern wird auf die Ausführungen zum Beispiel gemäß Fig. 3 Bezug genommen, die auch im Zusammenhang mit dem Beispiel gemäß Fig. 8 offenbart werden.

Die erste Temperierleitung 13a bzw. das zweite Arbeitslumen 13a stehen in distaler Richtung über das distale Ende der zweiten Temperierleitung 13b bzw. des dritten Arbeitslumens 13b vor. Der Ringspalt zwischen der ersten Temperierleitung 13a und der zweiten Temperierleitung 13b für den Rücklauf (siehe Pfeil in distaler Richtung) ist gut erkennbar. In der Wandung der ersten Temperierleitung 13a sind mehrere Austrittsöffnungen 20, insbesondere wenigstens eine Austrittsöffnung 20 vorgesehen, durch die das Temperiermedium aus der ersten Temperierleitung 13a ausströmen kann (siehe Pfeil). Die Austrittsöffnungen 20 sind im Bereich des distalen Endes der ersten Temperierleitung 13a angeordnet. Die Austrittsöffnungen 20 sind in Längsrichtung hintereinander angeordnet, wobei die am weitesten proximal angeordnete Austrittsöffnung 20 in etwa in der Mitte des vorstehenden Abschnittes der ersten Temperierleitung 13a vorgesehen ist.

Das proximale Ende des Temperierelements 12, konkret des Ballons 19 ist mit dem vorstehenden Teil der ersten Temperierleitung 13a, konkret mit dem distalen Ende der ersten Temperierleitung 13 fluiddicht verbunden. Das proximale Ende des Ballons 19 ist mit der zweiten Temperierleitung 13b fluiddicht, insbesondere mit einem distalen Ende der zweiten Temperierleitung 13b fluiddicht verbunden. Damit ist ein Aufnahmeraum durch den Ballon 19 aufgespannt, der sich entlang des vorstehenden Teils der ersten Temperierleitung 13a erstreckt und der von Temperiermittel durchströmbar ist.

Der distale Abschluss durch das Dichtelement 22 der ersten Temperierleitung 13a ist in Fig. 8 gut zu erkennen. Der Kanal 17 bzw. das erste Arbeitslumen 17 steht in distaler Richtung über das distale Ende der ersten Temperierleitung 13a vor. Es ist auch möglich, dass der Kanal 17 auf derselben Höhe wie die erste Temperierleitung 13a endet.

Es ist möglich, dass die Arbeitslumen 13a, 13b eine Querschnitsänderung in Längsrichtung des Katheters erfahren. Beispielsweise kann sich der Katheter in proximaler Richtung vergrößern, so dass die Druckverluste bei der Flüssigkeitszufuhr reduziert werden. Die weiter oben angegebenen Durchmesser beziehen sich auf den distalen Bereich des Katheters mit einer Länge von mindestens 10 mm, insbesondere mindestens 15 mm, insbesondere mindestens 20 mm, insbesondere mindestens 30 mm. Die Obergrenze des distalen Bereichs weist eine Länge von höchstens 60 mm, insbesondere höchstens 50 mm, insbesondere höchstens 40, insbesondere höchstens 30 mm auf. Die vorstehend genannten Ober- und Untergrenzen können zu Bereichen jeweils miteinander kombiniert werden.

Der vorstehend beschriebene Katheter gemäß Fig. 7 bis 9 wird im Zusammenhang mit einem System als Zufuhrlumen für einen Mikrokatheter oder einen Führungsdraht (Kanal 17) und als Einlasslumen und Auslasslumen für eine Kühlflüssigkeit offenbart und beansprucht, wobei sich die Kühlflüssigkeit im Einlasslumen in distaler und im Auslasslumen in proximaler Richtung bewegt.

Als Materialien können thermoplastische Kunststoffe, wie Pebax und PU mit oder ohne Metallverstärkung in Frage kommen.

Ferner wird ein System zum Temperieren, insbesondere Kühlen von Blut beschrieben, das einen mehrlumigen Katheter mit Temperierelement, wie vorstehend beschrieben, und eine Zuführleitung 18 im Kanal 17 aufweist. Im Unterschied zu den vorstehend genannten Beispielen weist das System keine Rekanalisationeinrichtung auf. Vielmehr ist die Zuführleitung 18 dazu angepasst, ein thrombenlösendes Medikament dem Blutgefäß zuzuführen und ist dazu mit einer geeigneten Medikamentenzufuhr verbunden.

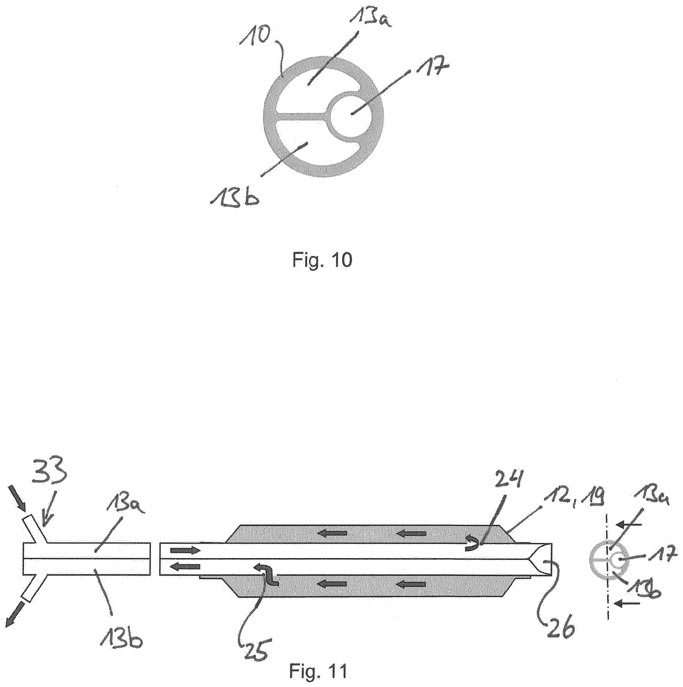

Eine weitere Zuführung 10, insbesondere Katheter, der für alle vorstehend beschriebenen Systeme geeignet ist und als solcher zusammen mit diesen offenbart und beansprucht wird, ist in den Figuren 10, 11 dargestellt.

Der Katheter 10 weist einen dreilumigen Querschnitt auf. Die beiden Temperierleitungen 13a, 13b sind im Querschnitt in etwa nierenförmig ausgebildet, und umgreifen teilweise den im Querschnitt kreisförmigen Kanal 17. Der Kanal 17 ist seitlich an der Wand des Katheters 10, d.h. exzentrisch im Katheter 10 angeordnet. Eine andere Aufteilung der Lumen ist möglich. Allgemein hat der Kanal 17 einen kleineren Querschnitt als jeweils eine Temperierleitung 13a, 13b.

Der Kanal 17 ist für die Zufuhr einer gesonderten Zufuhrleitung 18, insbesondere eines Mikrokatheters 18, insbesondere eines 2Fr Mikrokatheters geeignet. Der Kanal 17 hat vorzugsweise einen Durchmesser von 0,7 mm bis 1,1 mm, insbesondere von 0,8 mm bis 1,0 mm, insbesondere von 0,85 mm bis 0,95 mm.

Der Durchmesser des Katheters 10, an dem das Temperierelement 12, insbesondere der Temperierballon 19 angebracht ist, beträgt zumindest auf Höhe des Ballons von 2,0 mm bis 2,6 mm, insbesondere von 2,1 mm bis 2,5 mm, insbesondere von 2,2 mm bis 2,4 mm, insbesondere von 2,25 mm bis 2,35 mm.

Der Temperierballon 19 ist am distalen Ende des Katheters 10 angebracht. Vorzugsweise ist der Temperierballon 19 von der Spitze des Katheters 10 beabstandet. Der Abstand kann von 10 mm bis 30 mm betragen. An der Spitze des Katheters 10 kann ein Marker angeordnet sein (nicht dargestellt).

Die axiale Länge des Temperierballons 19 beträgt vorzugsweise von 3 cm bis 10 cm, insbesondere von 4 cm bis und 9 cm, insbesondere von 6 cm bis 8 cm. Der Durchmesser des expandierten Ballons 19 ist zwischen 4 mm und 8 mm, insbesondere zwischen 5 mm und 7 mm, insbesondere zwischen 5,5 mm und 6,5 mm. Die Länge, zumindest die implantierbare Länge des Katheters 10 ist zwischen 120 mm und 140 mm, insbesondere zwischen 125 und 135 mm. Der Mikrokatheter 18, der im Kanal 17 bewegt wird, hat einen distalen Durchmesser zwischen 0,55 und 0,7 mm, insbesondere zwischen 0,55 und 0,66 mm, wobei die letzteren Werte einem Bereich zwischen 1,7 und 2 French entsprechen.

Der Mikrokatheter 18 kann so aus dem Katheter 10 herausgeschoben werden, dass der distaler Bereich des Mikrokatheters 18 über den distalen Bereich des Kühlkatheters 10 vorsteht. Der vorstehende Bereich des Mikrokatheters 18 hat eine Länge von 10 cm bis 30 cm, insbesondere von 15 und 25 cm.

Der vorstehende Bereich entspricht dem maximal einstellbaren Abstand zwischen dem distalen Ende der Zuführleitung bzw. dem Mikrokatheter 18 und dem distalen Ende der Zuführung bzw. des Katheters 10, insbesondere des Kanals 17.

Wie in Figur 11 dargestellt, wird das Temperierelement 12, insbesondere der Temperierballon 19 durch die beiden Temperierleitungen 13a, 13b kontinuierlich mit Kühlmittel versorgt, wodurch die Kühlleistung verbessert wird. Konkret ist die zuführende Temperierleitung 13a durch eine Zufuhröffnung 24 mit dem Temperierelement 12 verbunden. Die Zufuhröffnung 24 ist distal angeordnet. Die abführende Temperierleitung 13b ist mit einer proximal angeordneten Abfuhröffnung 25 verbunden, so dass ein Erwärmen des im Temperierelement 12 strömenden Kühlmittels durch das abzuführende Kühlmittel vermieden wird. Hierbei sind jeweils eine einzige Zufuhr- und Abfuhröffnung 24, 25 dargestellt. Es ist auch möglich, jeweils mehrere Zufuhröffnungen 24 und Abfuhröffnungen 25 vorzusehen, beispielsweise auf dem Umfang verteilt angeordnete Öffnungen.

Allgemein sind in den Temperierleitungen 13a, 13b jeweils mindestens eine Zu- bzw. Abfuhröffnung 24, 25 ausgebildet, die die jeweilige Leitungen 13a, 13b mit dem Temperierelement 12 fluidverbindet. Im bevorzugten Fall ist eine einzige Öffnung 24, 25 pro Leitung 13a, 13b ausgebildet. Die Öffnungen 24, 25 sind relativ zur Achse des Temperierelementes 12, insbesondere des Temperierballons 19 voneinander versetzt, zB um 180° versetzt angeordnet. Die Öffnungen 24, 25 sind jeweils nah am jeweiligen Endbereich des Temperierballons 19 angeordnet. Es können auch mehrere Öffnungen jeweils für die Zufuhr und/oder die Abfuhr vorgesehen sein.

Am proximalen Ende des Katheters 10 ist gemäß Figur 11 ein Anschlusssystem 33 mit zwei Anschlüssen für das Kühlmittel vorgesehen, beispielsweise ein Luerkonnektor, durch den Kühlmittel zu- bzw. abgeführt werden kann.

Die beiden Temperierleitungen 13a, 13b sind distal von der Zuführöffnung 24 verschlossen, wie in Figur 11 zu erkennen. Dazu verjüngt sich die Wandung der beiden Temperierleitungen 13a, 13b und schließt mit der Außenwandung des Katheters 10 fluiddicht ab. Ein solcher Verschluss der beiden Temperierleitungen 13a, 13b wird in an sich bekannter Weise durch ein distal zugeführtes entsprechend profiliertes Werkzeug hergestellt, das die Wandungen der Temperierleitungen 13a, 13b verschweißt.

Die Spitze des Katheters 10 hat gemäß Fig. 11 einen Einführbereich 26. Alternativ können die beiden Temperierleitungen 13a, 13b durch einen Kleber bzw. Harz verschlossen sein. Die axialen Enden des Temperierballons 19 sind an dem Katheter 10 befestigt, zB verklebt oder lasergeschweisst.

Wenn als Behandlungseinrichtung eine Okklusionseinrichtung, konkret ein Aneurysmacoil verwendet wird, ist das Coil so angepasst, dass dieses nach dem Freisetzen aus dem Mikrokatheter 18 als expandiertes Gesamtgebilde einen Außendurchmesser aufweist, der größer als der Außendurchmesser des Mikrokatheters 18, insbesondere größer als der Außendurchmesser des Katheters 10 ist.

### Bezugszeichenliste

- 10: Zuführung / Katheter
- 11: Rekanalisationseinrichtung
- 12: Temperierelement
- 13a, b: Temperierleitungen
- 14: Proximales Ende des Temperierelements
- 15: Distales Ende des Temperierelements
- 16: Distales Ende der Temperierleitung
- 17: Kanal
- 18: Zuführleitung / Mikrokatheter
- 19: Temperierballon
- 20: Austrittsöffnung der Temperierleitung
- 21: Austrittsöffnung des Kanals
- 22: Dichtelement
- 23: Transportdraht
- 24: Zufuhröffnung
- 25: Abfuhröffnung

- BG: Blutgefäß
- T: Thrombus

## Patentansprüche

1. Medizinisches System zum endovaskulären Temperieren von Blut und zur Behandlung eines Blutgefäßes, insbesondere zur Rekanalisation eines Blutgefäßes, aufweisend
- eine Zuführung (10),
- eine radial komprimierbare Behandlungseinrichtung (11), insbesondere Rekanalisationseinrichtung (11), die im komprimierten Zustand in der Zuführung (10) längsbeweglich angeordnet und durch Entlassen aus der Zuführung (10) zur Behandlung des Blutgefäßes, insbesondere zur Rekanalisation des Blutgefäßes, radial expandierbar ist,
**dadurch gekennzeichnet, dass**
das System ein Temperierelement (12) zum Temperieren von Blut aufweist, wobei die Behandlungseinrichtung (11), insbesondere Rekanalisationseinrichtung (11), distal zum Temperierelement (12) positionierbar ist derart, dass im Gebrauch durch das Temperierelement (12) temperiertes Blut zur Behandlungsstelle, insbesondere zur Rekanalisationsstelle, im Blutgefäß strömt.

2. System nach Anspruch 1,
**dadurch gekennzeichnet, dass**
die Behandlungseinrichtung (11) einen Flow Diverter, eine Thrombektomieeinrichtung und eine Okklusionseinrichtung umfasst.

3. System nach Anspruch 1 oder 2,
**dadurch gekennzeichnet, dass**
die Behandlungseinrichtung (11), insbesondere Rekanalisationseinrichtung (11), eine radial expandierbare und radial komprimierbaren Gitterstruktur aufweist, die im expandierten Zustand von Blut durchströmbar ist.

4. System nach einem der Ansprüche 1 bis 3,
**dadurch gekennzeichnet, dass**
die Zuführung (10) einen Kanal (17) für die Behandlungseinrichtung (11), insbesondere Rekanalisationseinrichtung (11), aufweist, der sich entlang wenigstens einer Temperierleitung (13a, 13b) erstreckt, die von einem Temperiermedium durchströmbar und mit dem Temperierelement (12) verbunden ist.

5. System nach Anspruch 4,
**dadurch gekennzeichnet, dass**
eine Zuführleitung (18) im Kanal (17) längsbeweglich angeordnet ist, wobei die Behandlungseinrichtung (11), insbesondere Rekanalisationseinrichtung (11), in der Zuführleitung (18) längsbeweglich angeordnet ist.

6. System nach Anspruch 4,
**dadurch gekennzeichnet, dass**
die Behandlungseinrichtung (11), insbesondere Rekanalisationseinrichtung (11), unmittelbar im Kanal (17) längsbeweglich angeordnet ist.

7. System nach einem der Ansprüche 4-6,
**dadurch gekennzeichnet, dass**
wenigstens zwei Temperierleitungen (13a, 13b) für einen kontinuierlichen Vor- und Rücklauf des Temperiermediums mit dem Temperierelement (12) verbunden sind oder eine einzige Temperierleitung (13a) für einen pulsatilen Vor- und Rücklauf des Temperiermediums mit dem Temperierelement (12) verbunden ist.

8. System nach Anspruch 7,
**dadurch gekennzeichnet, dass**
die Temperierleitung (13a) für den Vorlauf innerhalb der Temperierleitung (13b) für den Rücklauf angeordnet ist.

9. System nach Anspruch 7 oder 8,
**dadurch gekennzeichnet, dass**
die Temperierleitung (13a) für den Vorlauf in distaler Richtung über die Temperierleitung (13b) für den Rücklauf vorsteht, wobei ein proximales Ende (14) des Temperierelements (12) mit der Temperierleitung (13b) für den Rücklauf und ein distales Ende (15) des Temperierelements (12) mit der Temperierleitung (13a) für den Vorlauf verbunden ist.

10. System nach Anspruch 9,
**dadurch gekennzeichnet, dass**
wenigstens eine Austrittsöffnung (20) in der Temperierleitung (13a) für den Vorlauf am distalen Ende (15) des Temperierelements (12) angeordnet ist.

11. System nach Anspruch 7,
**dadurch gekennzeichnet, dass**
der Kanal (17) in distaler Richtung über die einzige Temperierleitung (13a) für den Vor- und Rücklauf vorsteht, wobei ein proximales Ende (14) des Temperierelements (12) mit der einzigen Temperierleitung (13a) und ein distales Ende (15) des Temperierelements (12) mit dem Kanal (17) verbunden ist.

12. System nach einem der vorhergehenden Ansprüche,
**dadurch gekennzeichnet, dass**
das Temperierelement (12) einen Temperierballon, insbesondere einen profilierten Temperierballon (19) bildet.

13. System nach einem der Ansprüche 4-12,
**dadurch gekennzeichnet, dass**
die wenigstens eine Temperierleitung, insbesondere beide Temperierleitungen (13a, 13b), und der Kanal (17) für die Behandlungseinrichtung (11), insbesondere Rekanalisationseinrichtung (11), koaxial angeordnet sind.

14. Medizinisches System zum endovaskulären Temperieren von Blut aufweisend
- eine Zuführung (10), und
- ein Temperierelement (12) zum Temperieren von Blut,
**dadurch gekennzeichnet, dass**
das System eine Zuführleitung (18), insbesondere zur Verabreichung eines thrombolytischen Medikaments aufweist, die in der Zuführung (10) längsbeweglich angeordnet ist, wobei ein distaler Abschnitt der Zuführleitung (18) aus der Zuführung (10) heraus in distaler Richtung bewegbar ist,
wobei eine Austrittsöffnung des distalen Abschnitts der Zuführleitung (18) distal zum Temperierelement (12) positionierbar ist derart, dass im Gebrauch durch das Temperierelement (12) temperiertes Blut zur Austrittsöffnung des distalen Abschnitts der Zuführleitung (18) strömt.

15. System nach Anspruch 5 oder 14,
**dadurch gekennzeichnet, dass**
der maximal einstellbare Abstand zwischen dem distalen Ende der Zuführleitung (18) und dem distalen Ende der Zuführung (10), insbesondere des Kanals (17) von 10 cm bis 30 cm, insbesondere von 15 cm bis 25 cm beträgt.

## Claims

1. A medical system for endovascular temperature control of blood and for treatment of a blood vessel, in particular for recanalisation of a blood vessel, comprising
- a feed portion (10),
- a radially compressible treatment apparatus (11), in particular recanalisation apparatus (11), which, in a compressed state, is arranged in the feed portion (10) in a longitudinally movable manner and, by leaving the feed portion (10), can be radially expanded for treatment of the blood vessel, in particular for recanalisation of the blood vessel,
**characterised in that**
the system has a temperature control element (12) for controlling the temperature of blood, wherein the treatment apparatus (11), in particular recanalisation apparatus (11) can be positioned distally in relation to the temperature control element (12), in such a way that, during use, blood of which the temperature has been controlled by the temperature control element (12) flows towards the treatment site, in particular towards the recanalisation site, in the blood vessel.

2. The system according to claim 1,
**characterised in that**
the treatment apparatus (11) comprises a flow diverter, a thrombectomy apparatus, and an occlusion apparatus.

3. The system according to claim 1 or 2,
**characterised in that**
the treatment apparatus (11), in particular recanalisation apparatus (11), has a radially expandable and radially compressible mesh structure, through which blood can flow in the expanded state.

4. The system according to any one of claims 1 to 3,
**characterised in that**
the feed portion (10) has a channel (17) for the treatment apparatus (11), in particular recanalisation apparatus (11), which channel extends along at least one temperature control line (13a, 13b), through which a temperature control medium can flow and which is connected to the temperature control element (12).

5. The system according to claim 4,
**characterised in that**
a feed line (18) is arranged in the channel (17) in a longitudinally movable manner, wherein the treatment apparatus (11), in particular recanalisation apparatus (11), is arranged in the feed line (18) in a longitudinally movable manner.

6. The system according to claim 4,
**characterised in that**
the treatment apparatus (11), in particular recanalisation apparatus (11), is arranged directly in the channel (17) in a longitudinally movable manner.

7. The system according to any one of claims 4 - 6,
**characterised in that**
at least two temperature control lines (13a, 13b) are connected to the temperature control element (12) for continuous feed and return of the temperature control medium, or a single temperature control line (13a) is connected to the temperature control element (12) for a pulsatile feed and return of the temperature control medium.

8. The system according to claim 7,
**characterised in that**
the temperature control line (13a) for the feed is arranged inside the temperature control line (13b) for the return.

9. The system according to claim 7 or 8,
**characterised in that**
the temperature control line (13a) for the feed projects beyond the temperature control line (13b) for the return in the distal direction, wherein a proximal end (14) of the temperature control element (12) is connected to the temperature control line (13b) for the return and a distal end (15) of the temperature control element (12) is connected to the temperature control line (13a) for the feed.

10. The system according to claim 9,
**characterised in that**
at least one discharge opening (20)is arranged in the temperature control line (13a) for the feed at the distal end (15) of the temperature-control element (12).

11. The system according to claim 7,
**characterised in that**
the channel (17) projects beyond the single temperature control line (13a) for the feed and return in the distal direction, wherein a proximal end (14) of the temperature control element (12) is connected to the single temperature control line (13a) and a distal end (15) of the temperature control element (12) is connected to the channel (17).

12. The system according to any one of the preceding claims,
**characterised in that**
the temperature control element (12) forms a temperature control balloon, in particular a profiled temperature control balloon (19).

13. The system according to any one of claims 4 - 12,
**characterised in that**
the at least one temperature control line, in particular both temperature control lines (13a, 13b), and the channel (17) for the treatment apparatus (11), in particular recanalisation apparatus (11), are arranged coaxially.

14. A medical system for endovascular temperature control of blood, comprising
- a feed portion (10),
- a temperature control element (12) for controlling the temperature of blood,
**characterised in that**
the system has a feed line (18), in particular for administering a thrombolytic medicament, which feed line is arranged in the feed portion (10) in a longitudinally movable manner, wherein a distal portion of the feed line (18) can be moved out of the feed portion (10) in a distal direction,
wherein a discharge opening of the distal portion of the feed line (18) can be positioned distally in relation to the temperature control element (12), in such a way that, during use, blood of which the temperature has been controlled by the temperature control element (12) flows towards the discharge opening of the distal portion of the feed line (18).

15. The system according to claim 5 or 14,
**characterised in that**
the maximum distance than can be set between the distal end of the feed line (18) and the distal end of the feed portion (10), in particular of the channel (17), is from 10 cm to 30 cm, in particular from 15 cm to 25 cm.

## Revendications

1. Système médical pour la régulation endovasculaire de la température du sang et pour le traitement d'un vaisseau sanguin, en particulier pour la recanalisation d'un vaisseau sanguin, présentant
- une amenée (10),
- un dispositif de traitement (11) comprimable radialement, en particulier un dispositif de recanalisation (11) lequel, à l'état comprimé, est disposé de façon mobile longitudinalement dans l'amenée (10) et peut se dilater radialement par sortie hors de l'amenée (10) pour le traitement du vaisseau sanguin, en particulier pour la recanalisation du vaisseau sanguin,
**caractérisé en ce que**
le système présente un élément de régulation de température (12) pour réguler la température du sang, dans lequel le dispositif de traitement (11), en particulier un dispositif de recanalisation (11), peut être positionné de façon distale à l'élément de régulation de température (12) de manière à ce que, lors de l'utilisation par l'élément de régulation de température (12), du sang à température régulée s'écoule dans le vaisseau sanguin vers l'emplacement de traitement, en particulier vers l'emplacement de recanalisation.

2. Système selon la revendication 1,
**caractérisé en ce que**
le dispositif de traitement (11) comprend un déviateur de flux, un dispositif de thrombectomie et un dispositif d'occlusion.

3. Système selon la revendication 1 ou 2,
**caractérisé en ce que**
le dispositif de traitement (11), en particulier un dispositif de recanalisation (11), présente une structure grillagée dilatable radialement et comprimable radialement pouvant être traversée par du sang à l'état dilaté.

4. Système selon l'une des revendications 1 à 3,
**caractérisé en ce que**
l'amenée (10) présente un canal (17) pour le dispositif de traitement (11), en particulier un dispositif de recanalisation (11), lequel s'étend le long d'au moins une conduite de régulation de température (13a, 13b) qui peut être traversée par un milieu thermorégulateur et qui est reliée à l'élément de régulation de température (12).

5. Système selon la revendication 4,
**caractérisé en ce que**
une conduite d'amenée (18) est disposée de façon mobile longitudinalement dans le canal (17), dans lequel le dispositif de traitement (11), en particulier un dispositif de recanalisation (11), est disposé de façon mobile longitudinalement dans la conduite d'amenée (18).

6. Système selon la revendication 4,
**caractérisé en ce que**
le dispositif de traitement (11), en particulier un dispositif de recanalisation (11), est disposé directement dans le canal (17) de façon mobile longitudinalement.

7. Système selon l'une des revendications 4 - 6,
**caractérisé en ce que**
au moins deux conduites de régulation de température (13a, 13b) sont reliées à l'élément de régulation de température (12) pour l'aller et le retour continu du milieu thermorégulateur ou bien **en ce qu'**une seule conduite de régulation de température (13a) est reliée à l'élément de régulation de température (12) pour un aller et retour pulsatile du milieu thermorégulateur.

8. Système selon la revendication 7,
**caractérisé en ce que**
la conduite de régulation de température (13a) pour l'aller est disposée à l'intérieur de la conduite de régulation de température (13b) pour le retour.

9. Système selon la revendication 7 ou 8,
**caractérisé en ce que**
la conduite de régulation de température (13a) pour l'aller est en saillie en direction distale par rapport à la conduite de régulation de température (13b) pour le retour, dans lequel une extrémité proximale (14) de l'élément de régulation de température (12) est reliée à la conduite de régulation de température (13b) pour le retour, et une extrémité distale (15) de l'élément de régulation de température (12) étant reliée à la conduite de régulation de température (13a) pour l'aller.

10. Système selon la revendication 9,
**caractérisé en ce que**
au moins une ouverture de sortie (20) est disposée dans la conduite de régulation de température (13a) pour l'aller à l'extrémité distale (15) de l'élément de régulation de température (12).

11. Système selon la revendication 7,
**caractérisé en ce que**
le canal (17) est en saillie en direction distale par rapport à l'unique conduite de régulation de température (13a) pour l'aller et le retour, dans lequel une extrémité proximale (14) de l'élément de régulation de température (12) est reliée à l'unique conduite de régulation de température (13a), et une extrémité distale (15) de l'élément de régulation de température (12) étant reliée au canal (17).

12. Système selon l'une des revendications précédentes,
**caractérisé en ce que**
l'élément de régulation de température (12) est un ballon de régulation de température, en formant en particulier un ballon profilé de régulation de température (19).

13. Système selon l'une des revendications 4 - 12,
**caractérisé en ce que**
l'au moins une conduite de régulation de température, en particulier les deux conduites de régulation de température (13a, 13b), et le canal (17) pour le dispositif de traitement (11), en particulier un dispositif de recanalisation (11), sont disposés coaxialement.

14. Système médical pour la régulation endovasculaire de la température du sang, présentant
- une amenée (10) et
- un élément de régulation de température (12) pour réguler la température du sang,
**caractérisé en ce que**
le système présente une conduite d'amenée (18), en particulier pour administrer un médicament thrombolytique, laquelle est disposée de façon mobile longitudinalement dans l'amenée (10), dans lequel un tronçon distal de la conduite d'amenée (18) peut se déplacer en direction distale hors de l'amenée (10),
dans lequel une ouverture de sortie du tronçon distal de la conduite d'amenée (18) peut être positionnée de façon distale à l'élément de régulation de température (12) de manière à ce que, lors de l'utilisation, du sang à température régulée grâce à l'élément de régulation de température (12) circule vers l'ouverture de sortie du tronçon distal de la conduite d'amenée (18).

15. Système selon la revendication 5 ou 14,
**caractérisé en ce que**
la distance maximale réglable entre l'extrémité distale de la conduite d'amenée (18) et l'extrémité distale de l'amenée (10), en particulier du canal (17), est de 10 cm à 30 cm, en particulier de 15 cm à 25 cm.
